# EUROPEAN PATENT APPLICATION

(11) **EP 1 980 559 A1**
(43) Date of publication of application: **15.10.2008**
(21) Application number: 07425206.5
(22) Date of filing: 10.04.2007
(51) Int. Cl.: C07D 403/10, C07D 409/14, A61P 9/12, A61K 31/41, A61K 31/4184

(54) **Cardiovascular agents**

(71) Applicant: CTG Pharma S.r.l., 20131 Milan (IT)
(72) Inventor: Sparatore, Anna, 20146 Milan (IT); Santus, Giancarlo, 20146 Milan (IT); Del Soldato, Piero, 20052 Monza (MI) (IT)
(74) Representative: Nardini, Walter

(57) **Abstract**

The present invention relates to novel compounds that are derivatives of angiotensin receptor blocker (ARB) that comprise in their formula a polysulfurated group and that are useful for treating cardio-vascular diseases, such as hypertension, ischemic heart disease, atherosclerosis, metabolic syndrome, etc. also in combination with other cardiovascular agents.

## Description

### Field of the invention

This invention relates to the field of new compounds that are derivatives of angiotensin receptor blocker (ARB) whose main biological activity is in the cardiovascular area.

The angiotensin converting enzyme (ACE) inhibitors and the angiotensin receptor blocker (ARB) drugs both affect the renin-angiotensin hormonal system, which regulate the blood pressure. The ACE inhibitors work by blocking an enzyme that converts the inactive form of angiotensin (Ang I) in the blood to its active form. The active form of angiotensin (Ang II) constricts or narrows the arteries, but the inactive form cannot.

As an added benefit, ACE inhibitors may reduce an enlarged heart (left ventricular hypertrophy) in patients with hypertension. Moreover, they have been particularly useful in slowing the progression of kidney dysfunction in hypertensive patients with kidney disease resulting from Type 1 diabetes (insulin-dependent). Accordingly, ACE inhibitors usually are the first line drugs of choice to treat high blood pressure in cases that also involve congestive heart failure, chronic kidney failure in both diabetics and non-diabetics, and heart attack (myocardial infarction) that weakens the heart muscle (systolic dysfunction).

ACE inhibitors have few side effects. One bothersome side effect, however, is a chronic cough. For patients who develop a chronic cough on an ACE inhibitor, an ARB drug is a good substitute.

ARB drugs work by blocking the angiotensin receptor on the arteries to which activated angiotensin (Ang II) must bind to have its effects. As a result, the angiotensin is not able to work on the artery. The ARB drugs appear to have many of the same advantages as the ACE inhibitors but without the associated cough. Accordingly, they are also suitable as first line agents to treat hypertension. ARB drugs are currently recommended for first line renal protection in diabetic nephropathy (kidney disease).

ARB drugs include losartan (Cozaar^{®}) and its active metabolite EXP3174, irbesartan (Avapro^{®}) valsartan (Diovan^{®}), candesartan (Atacand^{®}), olmesartan (Benicar^{®} telmisartan (Micardis^{®}), and eprosartan (Teveten^{®}).

Their adverse effects are similar to those described for ACE inhibitors, including the hazard of use in pregnancy. Cough and angioedema can occur but are less common. It should be also outlined that politherapy of hypertension treatment is popular, because each of the drugs acts on one of a set of compensatory regulatory mechanisms for maintaining blood pressure. But polipharmacy has also limitations because compliance can be worse. Thus a still unmet medical need is to discover new, more potent and/or safer anti-hypertensive agents.

The relationship of NADPH oxidase (NOX) with Ang II is of particular pathogenetic significance and its interaction has relevant vasomotor consequences.

NOX plays a central role on the complex interaction at the vessel wall between oxidative stress and inflammation leading to endothelium dysfunction. [H. Cai, G. Griendling, D. Harrison; The vascular NAD(P)H oxidases as therapeutic targets in cardiovascular diseases. *TRENDS in Pharmacological Sciences* 2003; **24**: 471-478]. Oxidative stress comes from redox imbalance between NOX, a major source of free radicals in the vascular cell and anti-oxidative enzymes. In these conditions NOX is over-expressed and interacts significantly with Ang II, and other receptors.

New compounds, that are NOX hybrid inhibitors with additional Ang II inhibitory properties, are described in the present invention.

### Summary of the invention

This invention relates to novel ARB hybrids derivatives compounds that comprise in their formula a polysulfurated group and that are useful for treating diseases and pathological conditions involving the cardiovascular system. The results have been surprising, in that not only the safety was dramatically improved but also the efficacy was sometimes increased as compared with known ARB.

This invention also relates to processes for preparing these compounds and to pharmaceutical compositions containing these compounds.

The polysulfurated groups object of the present invention contain 2 or more atom of sulphur. The polysulfurated groups of the present invention linked to the ARB compounds are organic thiosulfonates or dithiole-thione derivatives such as 5-(4-hydroxyphenyl)-3H-1,2-dithiol-3-thione, or 4-(4-hydroxyphenyl)-3H-1,2-dithiol-3-thione, or 4-(3-thioxo-3H-1,2-dithiol-4-yl)benzoic acid, or 4-(3-thioxo-3H-1,2-dithiol-5-yl)benzoic acid, 1,3-dithiol-2-thione-5-carboxylic acid, 3-thioxo-3H-1,2-dithiole-5-carboxylic acid, 3-thioxo-3H-1,2-dithiole-4-carboxylic acid.

### Description of the invention

An object of the present invention are new ARB derivative compounds of general formula:

A-Y-W (I)

wherein
A is selected from the group comprising the ARB residues of formula (II) or (III) or (IV): 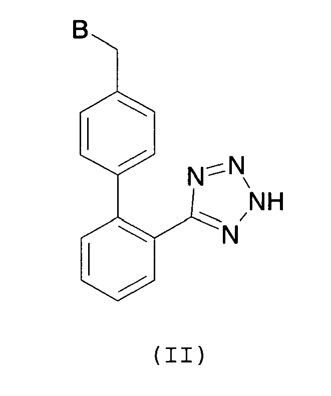 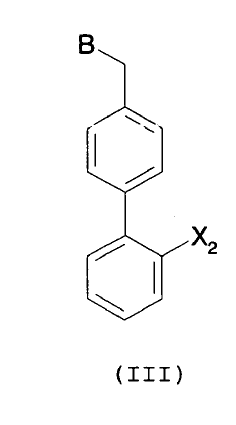 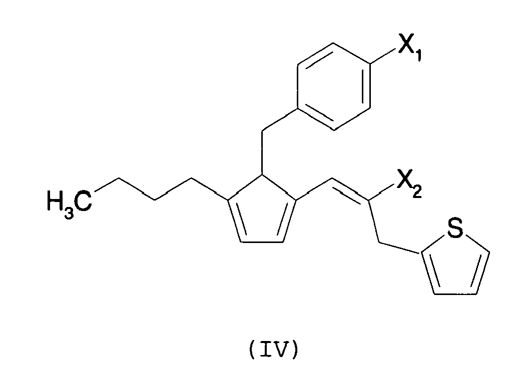 B is selected from the group comprising the residues of formula (IIa) or (IIb) or (IIc) when A has formula (II) or (III): 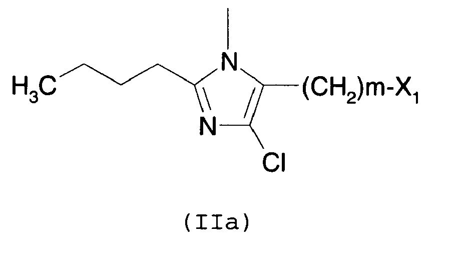 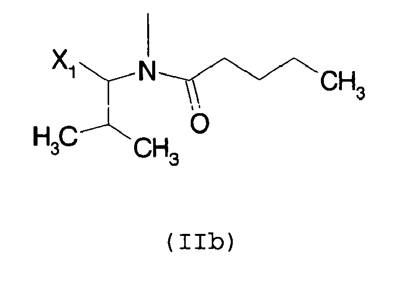 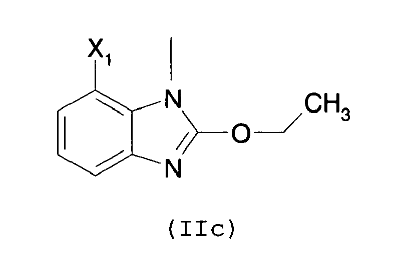 or B is the residue of formula (IIIa) when A has formula (III): 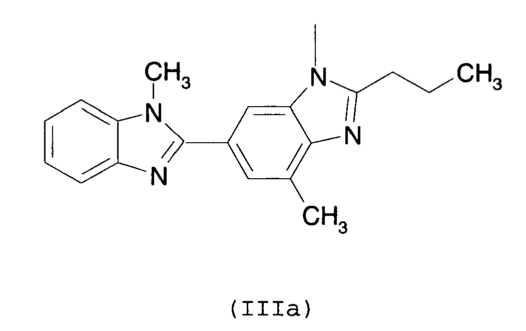 wherein both X₁ and X₂, the same or different to each other, are a group capable to bind to -Y or -W, selected from the group comprising -COO~; -O~; -CONH~; -OCO~; -OCOO~; -CO~;
and wherein X₁ is also, alternatively, -COOH or -H when X₂ is a group capable to bind to -Y or -W, selected from the group comprising -COO~; -O~; -CONH~; -OCO~; -OCOO~; -CO~;
and wherein X₂ is also, alternatively, -COOH or -H when X₁ is a group capable to bind to ~Y or ~W, selected from the group comprising -COO~; -O~; - CONH~; -OCO~; -OCOO~; -CO~;
Y is zero; ~ (Cₙ,) alkyl-, ~(C_{n'})alkyl-CO-, ~O-(C_{n'})alkyl-O-, ~OOC-(C_{n'}) alkyl-COO- ; ~O-(C_{n'})alkyl-, ~HN-(C_{n'})alkyl-, ~OOC-(C_{n'})alkyl-; ~(C_{n'})alkyl-O-CO-(C_{n''}) alkyl-; ~(C_{n'}) alkyl-CO-O-(C_{n''}) alkyl- wherein (C_{n'})alkyl and (C_{n''})alkyl are straight or branched, and n' and n" , the same or different to each other, are 0-10;
m is 0, 1-10;
W is a polysulfurated group containing 2 or more atoms of sulphur, selected from the group comprising an organic thiosulfonate moiety or a dithiole-thione derivative:
more in particular, as a further preferred embodiment, W is an organic thiosulfonate moiety of formula:

~S-SO₂-R (V)

wherein ~S-SO₂-R is linked to A-Y~; R is a straight or branched alkyl, such as methyl, ethyl, propyl; alkenyl, alkinyl; alkylaryl, alkenylaryl, alkinylaryl; arylalkyl, arylalkenyl, arylalkinyl; or cycloalkyl, cycloalkenyl, cycloalkinyl; or aromatic and/or heterocyclic ring, all substituted or unsubstituted;
or more in particular, as a further preferred embodiment, W is a dithiole-thione derivative of formula: 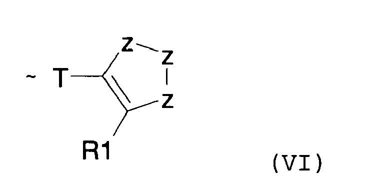 wherein
Z is S (sulphur) and at least 1 Z is C=S (thione), and
T is:
-OOC-; or 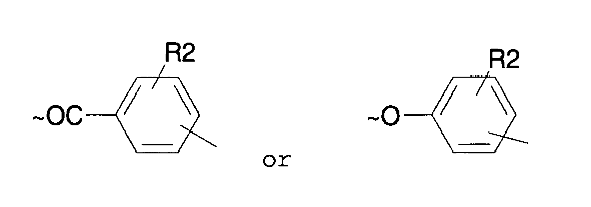 wherein
R1 is H; -COOH; -NH₂; -OH; -SH;
R2 is hydrogen; -COOH; alkyl, alkenyl, alkynyl; aryl; fluoro, chloro, bromo; hydroxyl, alkyloxy, alkenyloxy, aryloxy, acyloxy; amino, alkylamino, arylamino; thio; cyano; nitro; acyl; amido; and a 5 or 6-membered aromatic or non-aromatic ring containing 0, 1, or 2 heteroatoms selected from nitrogen; oxygen; or sulphur;
and salts thereof.
As a further preferred embodiment of the compounds according to general formula (I) of the present invention (Cₙ)alkyl, (C_{n'})alkyl and (C_{n''})alkyl are (CH₂)_{nA} , (CH₂)_{nA'} , (CH₂)_{nA''} respectively, wherein nA, nA' and nA", the same or different to each other, are 1-10, such as that more preferably Y is selected from the group comprising -(CH₂)_{nA'}-, ~ (CH₂)_{nA'}-CO-, ~O-(CH₂)_{nA'}-O-, ~OOC-(CH₂)_{nA'}-COO- ; ~O-(CH₂)_{nA'}-, -HN-(CH₂)_{nA'}-, ~OOC- (CH₂) _{nA},-; ~ (CH₂)_{nA'}-O-CO- (CH₂) _{nA''}-; ~(CH₂)_{nA'}-CO-O-(CH₂)_{nA''}- wherein nA, nA' and nA", the same or different to each other, are 1-10.

As a further preferred embodiment of the ARB derivative compounds according to the present invention, are the compounds of general formula (I) wherein the group -Y-W is selected from the group comprising thiosulfonate moieties derived from the corresponding precursor having the formula: S-(2-carboxyethyl)methanethiosulfonate, S-(2-aminoethyl)methanethiosulfonate and S-(2-hydroxyethyl)methanethiosulfonate.

As a further preferred embodiment of the ARB derivative compounds according to the present invention, are the compounds of general formula (I) wherein the polysulfurated group W is selected from the group comprising dithiole-thione derivatives of the corresponding precursors having the formula: 5-(p-hydroxyphenyl)-3H-1,2-dithiol-3-thione, 1,3-dithiol-2-thione-5-carboxylic acid, 3-thioxo-3H-1,2-dithiole-5-carboxylic acid, 3-thioxo-3H-1,2-dithiole-4-carboxylic acid.

The ARB compounds, in particular those selected from the group comprising losartan and its active metabolite EXP3174, irbesartan, valsartan, candesartan, olmesartan, telmisartan and eprosartan, and the moiety containing the polysulfurated group can be linked via different linking groups such as esters, amides, imides, sulfonamides, azo groups, carbamates, carbonates, anhydrides, acetals, thioacetals, etc. The polysulfurated group, i.e. the thiosulfonate moiety or dithiol-thione derivative can be also directly linked by an ionic bond to the ARB as salt when Y=0.

Bi-functional linkers (Y) known to the expert in the field (such as ethyl, propyl, or butyl diols; diamines; hydroxy amines, etc.) can be optionally present when they are necessary to link the drug (ARB compounds) to the polysulfurated group.

As a further object of the present invention are the preferred compounds according to general formula (I) such as:
4-(3-thioxo-3*H*-1,2-dithiol-4-yl)benzoic acid 2-butyl-4-chloro-1-[p-(o-1H-tetrazol-5-ylphenyl)-benzyl]imidazole-5-methyl ester 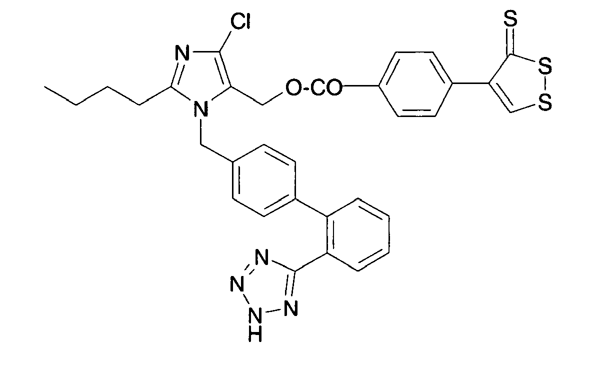
2-butyl-4-chloro-1-[(2'-(1H-tetrazol-5-yl)(1,1'-biphenyl)-4-yl)methyl]-1H-imidazole-5-carboxylic acid 4-(3H-1,2-dithiole-3-thione-5-yl)-phenyl ester 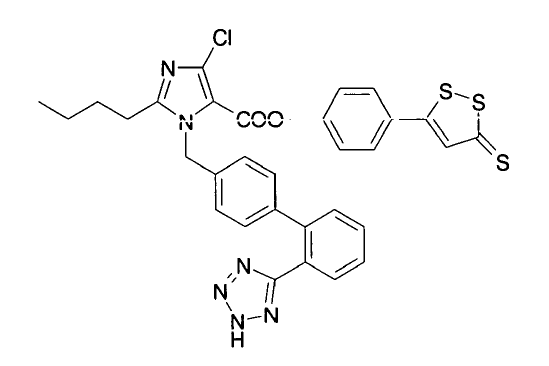
2-butyl-4-chloro-1-[(2'-(1H-tetrazol-5-yl)(1,1'-biphenyl)-4-yl)methyl]-1H-imidazole-5-carboxylic acid 2-(methylsulfonylthio)ethyl ester 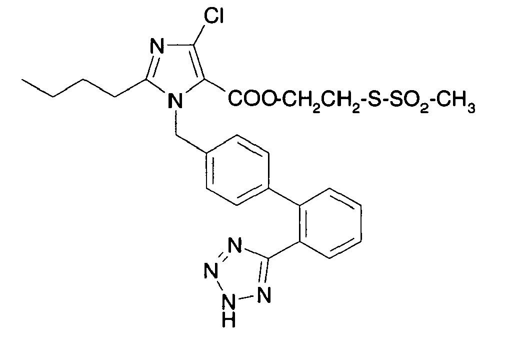
3-(methanesulfonylsulfanyl)-propionic acid 2-butyl-4-chloro-1-[*p*-(*o*-1*H*-tetrazol-5-ylphenyl)benzyl]-imidazole-5-methyl ester 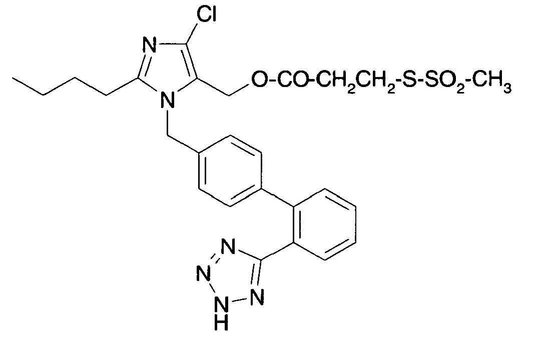
4'-[(1,4'-dimethyl-2'-propyl[2,6'-bi-1*H-*benzimidazol]-1' yl)methyl][1,1'-biphenyl]-2-carboxylic acid 4-(3H-1,2-dithiole-3-thione-5-yl)-phenyl ester

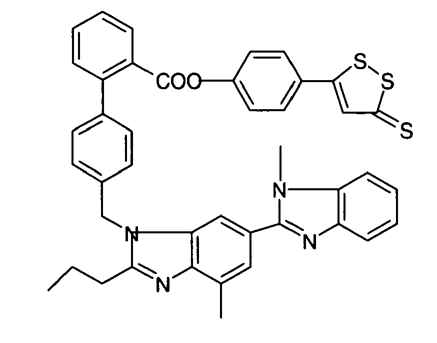

When the compounds include at least one asymmetric carbon atom, the products can be used in racemic mixture or in form of single enantiomer.

In the present invention the parent compound is considered in its original form or in a proper modification to allow the chemical manipulation with the polysulfurated group.

It is a further object of the present invention the pharmaceutical acceptable salts of compounds of formula (I), such as for example salts with alkaline metals and alkaline earth metals, non-toxic amines and aminoacids, inorganic acids such as hydrochloric acid, phosphoric acid, etc. or organic acids such as fumaric acid, citric acid, tartaric acid, etc.

Salts of organic thiosulfonates such as, for example, S-(2-carboxyethyl)methanthiosulfonate, S-(2-aminoethyl)methanthiosulfonate with the different ARB derivatives above described, are also part of the present invention. Salts of dithiolthiones such as, for example, 1,3-dithiol-2-thione-5-carboxylic acid, 3-thioxo-3H-1,2-dithiole-5-carboxylic acid, 3-thioxo-3H-1,2-dithiole-4-carboxylic acid with the different ARB derivatives above described are also part of the present invention.

According to the present invention it has been found that it is possible to link an organic polysulfurated group to an ARB drug for treating cardiovascular diseases. The resulting compounds have good bioavailability, increased safety and maintain good efficacy.

The main advantages of the compounds of the present invention are related to their biological activity for the treatment of patients with redox-based cardio-vascular diseases, such as hypertension, ischemic heart disease, atherosclerosis, metabolic syndrome, etc.

As a further object of the present invention are pharmaceutical compositions comprising at least one compound of the above said ARB derivative compounds (according to the present invention as for general formula (I), and the preferred compounds as described above) including salts thereof, as an active ingredient, moreover, as a further object of the present invention, in combination with pharmaceutically acceptable adjuvant(s) or carrier(s).

It is a further object of the present invention the use of the above said ARB derivative compounds as for general formula (I), and the preferred compounds as described above, as a medicament.

As a further object of the present invention is the use of compounds according to the present invention, as for general formula (I) and the preferred compounds as described above, for the preparation of a pharmaceutical composition, and therefore the corresponding method, for treating cardio-vascular diseases, such as hypertension, ischemic heart disease, atherosclerosis, metabolic syndrome, etc. also in combination with other cardiovascular agents.

It is a further object of the present invention the process of the synthesis of the above said ARB derivative compounds (according to the present invention as for general formula (I), and the preferred compounds as described above) including salts thereof, said process comprising at least a reaction between the corresponding precursor of an organic thiosulfonate or dithiol-thione, moiety W or Y-W, in particular selected from the group comprising: S-(2-carboxyethyl)methanethiosulfonate, S-(2-aminoethyl)methanethiosulfonate and S-(2-hydroxyethyl)methanethiosulfonate or 5-(p-hydroxyphenyl)-3H-1,2-dithiol-3-thione, 1,3-dithiol-2-thione-5-carboxylic acid, 3-thioxo-3H-1,2-dithiole-5-carboxylic acid, 3-thioxo-3H-1,2-dithiole-4-carboxylic acid and an ARB compound, in particular selected from the group comprising losartan and its active metabolite EXP3174, irbesartan, valsartan, candesartan, olmesartan, telmisartan, and eprosartan or at least a reaction between the corresponding precursor of an organic thiosulfonate or dithiol-thione, moiety W and a corresponding precursor of an ARB modified with Y, wherein W and Y have the above said meaning,

The compounds of the present invention can be administered in the form of any pharmaceutical formulation, the nature of which will depend upon the route of administration and the nature of the disease to be treated. These pharmaceutical compositions can be prepared by conventional methods, using compatible, pharmaceutically acceptable excipients or vehicles.

Examples of such compositions include capsules, tablets, syrups, powders and granulates for the preparation of extemporaneous solutions, injectable preparations, rectal, nasal, ocular, vaginal etc. A preferred route of administration is the oral route.

The following non-limitative examples further describe and enable an ordinary skilled in the art to make and use the invention.

### EXAMPLE 1. Synthesis of 4-(3-thioxo-3H-1,2-dithiol-4-yl)benzoic acid 2-butyl-4-chloro-1-[p-(o-1H-tetrazol-5-ylphenyl)-benzyl]imidazole-5-methyl ester

### Step 1: Preparation of 4-(3-thioxo-3H-1,2-dithiol-4-yl)benzoic acid

4-Isopropylbenzoic acid (1.0 g; 6.09 mmol) was suspended in 25 ml of ethanol and to this suspension 0.140 g of H₂SO₄ conc were added. The reaction was performed at 100° C, stirring for 9 hours.

The solution was evaporated to dryness and the residue was dissolved in CH₂Cl₂. The organic phase was washed with a saturated NaHCO₃ solution and then with brine, dried on anhydrous sodium sulphate and finally evaporated to dryness to obtain an oily colourless product.

This product (ethyl 4-isopropyl benzoate,940 mg; 5.16 mmol) was added dropwise to stirred melted sulfur (1.2 g) at 146°C and the reaction mixture was stirred at 220°C for 24 hours. The temperature is lowered to 110 °C and 3 ml of toluene and 7 ml of acetone were added. After sirring the reaction mixture at r.t. for 4 h, unreacted sulphur was filtered and the obtained solution was evaporated to dryness. The residue was purified by column chromatography on silica gel, eluting with CH₂C1₂- cyclohexane (6:4) to give a compound with m.p. 157.5-159.5°C. Finally a suspension of this compound (100 mg, 0.35 mmol) in 4.5 ml of acetic acid and 0.72 ml of 9M H₂SO₄ was stirred at 100°C for 4 hours. After cooling, the solution was diluted with water and extracted with a mixture of CH₂Cl₂-methanol (9:1). The organic phase was dried on anhydrous sodium sulphate and evaporated to dryness and the residue was washed with ether and CH₂C1₂ to obtain a yellow- orange solid, 4-(3-thioxo-3H-1,2-dithiol-4-yl)benzoic acid, with m.p. 240-245 °C.

### Step 2:

A solution of dicyclohexylcarbodiimide (DCC) (463mg; 1.2 eq.) in anhydrous tetrahydrofurane (THF) was added dropwise to a solution of losartan (800 mg; 1.67 mmol), 4-(3-thioxo-3H-1,2-dithiol-4-yl)benzoic acid (475.6 mg; 1.87 mmol) prepared as above described and dimethylaminopyridine (DMAP, 13 mg) in 19 ml of anhydrous THF. The reaction was performed at room temperature, stirring under nitrogen for 5 hours. At the end of the reaction, the dicyclohexylurea (DCU) was removed by filtration. The solution was evaporated to dryness and the residue was chromatographed on a silica gel column, eluting with a mixture of CH₂Cl₂-methanol (98:2) to give a compound with m.p. 218-222 °C.

### EXAMPLE 2. Synthesis of 2-butyl-4-chloro-1-[(2'-(1H-tetrazol-5-yl) (1,1'-biphenyl)-4-yl)methyl]-1H-imidazole-5-carboxylic acid 4-(3H-1,2-dithiole-3-thione-5-yl)-phenyl ester

### Step 1: Preparation of 5-(4-hydroxyphenyl)-3H-1,2-dithiole-3-thione

To 280 mmol of sulphur, 40 mmol of anethole in 20 ml of dimethylacetamide were added. After heating at 145 °C for 6 hours, 2.5 g of anethole dithiolethione (ADT) were obtained. The product, washed with ether, was crystallized by ethyl acetate: melting point 110-111 °C. Then 1.5g of ADT were mixed with 7.5g of pyridine HCl and the mixture was heated for 25 minutes at 215 °C. After cooling, 1N HC1 in excess was added and the precipitate was filtered, washed and crystallized from ethanol. The obtained compound, 5-(p-hydroxyphenyl)-3H-1,2-ditiol-3-thione, melted at 191-192 °C.

### Step 2: Preparation of 2-butyl-4-chloro-1-[(2'-(1-H-tetrazol-5-yl)biphenyl-4-yl)methyl]-imidazole-5-carboxylic acid

To a solution of 1.197 g of KOH (86%) in 15 ml of H₂O cooled at 2 °C, losartan potassium (700 mg), commercially available, was added. After stirring the mixture for 2 hours, sodium periodate (NaIO₄, 775 mg) followed by ruthenium (III) chloride hydrate (RuC1₃.H₂0, 16.9 mg) was added and the resulting slurry was stirred at 4-6 °C for 19 hours under nitrogen. At the end of the reaction, 0.13 ml of isopropyl alcohol were added and the mixture was stirred, at room temperature, for 3 hours. After filtration, 1.68 ml of phosphoric acid were added to the solution and the obtained suspension was stirred for 30 min and then filtered. The residue, washed with water, was dissolved in methanol and decolorized with activated charcoal and finally, after evaporation of the solvent, was washed with ether to yield a white solid with m.p. 176-178 °C.

### Step 3:

A solution of dicyclohexylcarbodiimide (DCC) (267.4 mg; 1.3 eq.) in anhydrous THF (10 ml) was added dropwise to a stirred solution of losartan acid (472 mg; 1.08 mmol) prepared as described in step 2, 5-(4-hydroxyphenyl)-3H-1,2-dithiol-3-thione (244.5 mg; 1.08 mmol) prepared as above described (step 1) and dimethylaminopyridine (DMAP, 7 mg) in 15 ml of tetrahydrofurane (THF).

The reaction is performed at room temperature, stirring under nitrogen for 5 hours. At the end of the reaction the dicyclohexylurea (DCC) is removed by filtration. The solution is evaporated to dryness and the crude product was purified by column chromatography (on silica gel) eluting with CH₂C1₂-methanol (97:3) to give an orange solid with melting point 180-185 °C.

### EXAMPLE 3. Synthesis of 2-butyl-4-chloro-1-[(2'-(1H-tetrazol-5-yl)(1,1-biphenyl)-4-yl)methyl]-1H-imidazole-5-carboxylic acid 2-(methylsulfonylthio)-ethyl ester

### Step 1: Synthesis of methanethiosulfonic acid S-(2-hydroxyethyl) ester

A solution of CH₃SO₂Cl (5.9 g) in ethanol (9.2 ml) is added dropwise to a refrigerated (-15°C) solution of Na₂S (46.98 mmol) in ethanol(34.5 ml).

The reaction mixture is stirred at room temperature for 12 hours. After filtration and crystallization from ethanol, sodium methanthiosulfonate, as a white solid is obtained. The sodium methanthiosulfonate (2.5 g; 18.64 mmol) is dissolved in 30 ml of ethanol and a solution of 2-bromoethanol (2.6 ml; 37.28 mmol) in ethanol (6 ml) is added dropwise. The solution is heated at 100 °C for 8 hours under nitrogen. The mixture is filtered, the solution is evaporated to dryness and the residue is dissolved in CHCl₃ and extracted with water.

The aqueous solution is evaporated to dryness, tetrahydrofuran (THF) is added to the residue and the obtained suspension is filtered. The THF solution is evaporated and methanethiosulfonic acid S-(2-hydroxyethyl) ester as an oily yellow product is obtained.

### Step 2:

A solution of dicyclohexylcarbodiimide (DCC) (267.4 mg; 1.3 eq.) in anhydrous THF (10 ml) was added dropwise to a stirred solution of losartan acid (472 mg; 1.08 mmol) prepared as described in EXAMPLE 2, step 2, methanethiosulfonic acid S-(2-hydroxyethyl) ester (1.08 mmol) prepared as above described (step 1) and dimethylaminopyridine (DMAP, 7 mg) in 15 ml of tetrahydrofurane (THF). The reaction is performed at room temperature, stirring under nitrogen for 5 hours.

At the end of the reaction the dicyclohexylurea (DCC) is removed by filtration. The solution is evaporated to dryness and the crude product was purified by column chromatography (on silica gel) eluting with CH₂Cl₂-methanol (98:2) to give the desired product with a melting point of 105-110 °C.

### EXAMPLE 4: Synthesis of 3-(methanesulfonylsulfanyl)-propionic acid 2-butyl-4-chloro-1-[p-(o-1H-tetrazol-5-ylphenyl)-benzyl]imidazole-5-methyl ester

### Step 1: Synthesis of 3-methanesulfonylsulfanyl-propionic acid

Sodium methanthiosulfonate (1.5 g; 11.18 mmol) and 3-bromopropionic acid (900 mg; 5.88 mmol) 97% are dissolved in dimethylformamide (DMF, 9.05 ml). The reaction is performed at 60° C, stirring under nitrogen for 4 hours.

The solution is evaporated to dryness and the residue is dissolved in water, acidified with 10 ml of a 2N KHSO₄ and washed with ethyl acetate. The organic phase is extracted with cold 2N KHSO₄, then with brine and finally dried on anhydrous sodium sulphate and evaporated to dryness. An oily yellow product is obtained.

### Step 2:

A solution of dicyclohexylcarbodiimide (DCC) (463mg; 1.2 eq.) in anhydrous tetrahydrofurane (THF) was added dropwise to a solution of losartan (800 mg; 1.67 mmol), 3-methanesulfonylsulfanyl-propionic acid (1.87 mmol) prepared as above described and dimethylaminopyridine (DMAP, 13 mg) in 19 ml of anhydrous THF.
The reaction was performed at room temperature, stirring under nitrogen for 5 hours.
At the end of the reaction, the dicyclohexylurea (DCU) was removed by filtration. The solution was evaporated to dryness and the residue was chromatographed on a silica gel column, eluting with a mixture of CH₂Cl₂-methanol (98:2) to give the desired compound.

### EXAMPLE 5 Synthesis of 4'-[(1,4'-dimethyl-2'-propyl[2,6'-bi-1H-benzimidazol]-1'-yl)methyl][1,1'-biphenyl]-2-carboxylic acid 4-(3H-1,2-dithiole-3-thione-5-yl)-phenyl ester

1-(3-dimethylaminopropyl)-3-ethylcarbodiimide hydrochloride (EDAC, 31.5 mg, 0.165 mmol) is added to a stirred, cold (0°C) solution of telmisartan (78 mg, 0.15 mmol), 5-(p-hydroxyphenyl)-3H-1,2-ditiol-3-thione (36 mg, 0.16 mmol), prepared as described in example 2 step 1, and dimethylaminopyridine (DMAP, 1 mg) in CHCl₃ (6 ml). The mixture is stirred at room temperature, under nitrogen for 20 h.

The chloroformic solution is extracted with water (3 times), dried with anhydrous sodium sulphate and evaporated to dryness.

After washing the residue with ether and chromatography on silica with CH₂Cl₂-MeOH (98.5-1.5), the obtained compound is an orange powder, with m.p. 180.5 - 182 °C.

### EXAMPLE 6. NAD(P)H oxidases inhibition

The method described by Muzaffar S, Shukla N, Angelini GD, Jeremy JY; "Prednisolone augments superoxide formation in porcine pulmonary artery endothelial cells through differential effects on the expression of nitric oxide synthase and NADPH oxidase" in Br. J. Pharmacol. 2005 Ju1;145(5):688-97 was used.

Briefly lungs were obtained from male pigs of body weight ranging from 20 - 35 kg. Pulmonary arteries (PA) of between 3-4 mm diameter were dissected from the lungs within 30 minutes and placed in Dulbecco's Minimum Essential Medium (DMEM). The arteries were then cut into 2 mm segments for preparation of cell cultures. PAECs were grown in Endothelial Cell Growth Medium at 37°C in a 95% air-5% CO₂ incubator. The measurement of superoxide (O₂⁻) release by cells was performed by detection of ferricytochrome c reduction.

The reduction of cytochrome c that was inhibitable with SOD reflected actual O₂⁻ release. PAVSMCs and PAECS were seeded and incubated with the TXA₂ analogue, U46619, isoprostane F_{2α},LPS (endotoxin) human recombinant IL-1α and human recombinant TNF-α, alone or with drugs for 16 hours. RESULTS

LPS, IL-1α, TNF-α and U46619 all promoted the formation of O₂⁻ in both porcine pulmonary artery VSMCs (PAVSMCs) and endothelial cells (PAECS) compared to untreated controls following a 16 hour incubation. In previous studies we established that this was due to an upregulation of NADPH oxidase expression and activity.

The test compounds (all 100 nM) inhibited the formation of O₂⁻ in both PAVSMCs and PAECs when stimulated with LPS, TNFα and IL1α. Losartan at similar concentration had no significant effect.

The test compounds (all 100 nM) inhibited the expression of gp91^{phox} when stimulated with TNFα in PAVSMCs when stimulated with LPS, TNFα and IL1α. Losartan at similar concentration had no significant effect. Identical results were obtained when gp91^{phox} upregulation was elicited with interleukin and LPS.

### EXAMPLE 7. Study on Vascular Tone

The ability of ARB derivatives to induce vasorelaxation in comparison to native ARB, was tested in vitro in isolated rabbit thoracic aorta preparations (Wanstall J. C. et al., Br. J. Pharmacol., 134:463-472, 2001). Male New Zealand rabbits were anaesthetized with thiopental-Na (50 mg/kg, iv), sacrificed by exsanguinations and then the thorax was opened and the aorta dissected.

Single ring preparations (4 mm in length) of thoracic aorta were set up in physiological salt solution at 37 °C in small organ chambers (5 ml). Each ring was mounted under 2g passive tension in 5 ml organ bath. Preparations were allowed to equilibrate for 1 h, then contracted submaximally with noradrenaline (NA, 1 µM) and, when the contraction was stable, acetylcholine (ACh, 10 µM) was added.

A relaxant response to ACh indicated the presence of a functional endothelium.

Under these experimental conditions, the native ARB, losartan, did not produce relaxation at any of the concentration tested, the curve being not different from that built up in presence of vehicle alone. On the contrary the ARB derivatives of the present invention were able to induce relaxation in a concentration-dependent manner.

### EXAMPLE 8. Antihypertensive activity in vivo

The ability of compound of example 1 to decrease blood pressure was evaluated in conscious spontaneously hypertensive rats (SHRs).

Three groups of SHRs (250-300g) received daily oral dose of losartan (10 mg/kg po) or compound of example 1 (equimolar dose) or vehicle (SHR control group) for 3 days. Systolic blood pressure (SBP) and heart rate were monitored by telemetry at different time points after dosing. The SBP was measured at baseline and after 1 hour, 12 hours and 24 hours.

The results of SBPs are reported in the following table:

**Table I: Systolic blood pressure in SHRs**

| Systolic blood pressure (SBP) mm Hg | | | | |
|---|---|---|---|---|
| Compound | Baseline | 1 hr | 12 hr | 24 hr |
| Vehicle | 190 | 187 | 186 | 191 |
| Losartan | 143 | 135 | 134 | 135 |
| Cpd EX.1 | 145 | 120 | 121 | 122 |

As reported in table I, the compounds of the present invention are able to reduce blood pressure in a more effectively way than the parent compound.

## Claims

1. Compounds of general formula:
A-Y-W (I)
wherein
A is selected from the group comprising the ARB residues of formula (II) or (III) or (IV): 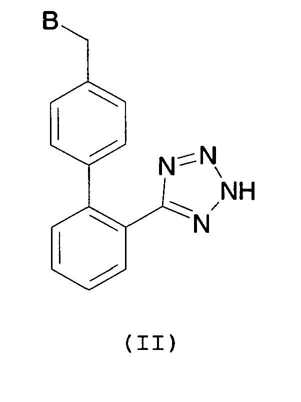 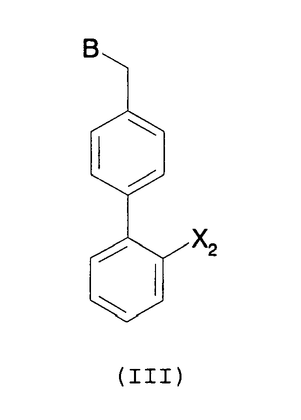 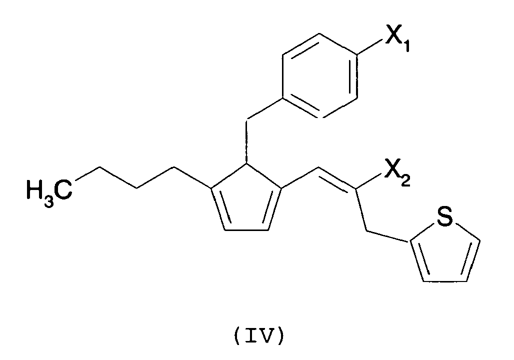 B is selected from the group comprising the residues of formula (IIa) or (IIb) or (IIc) when A has formula (II) or (III): 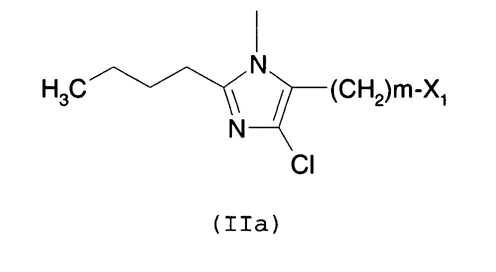 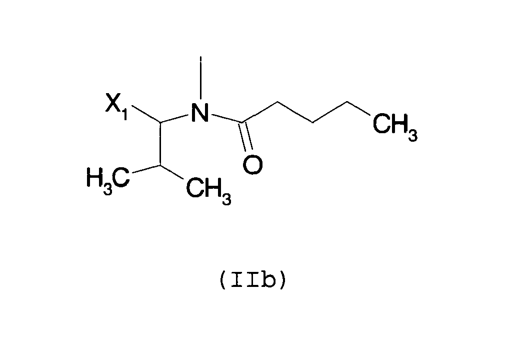 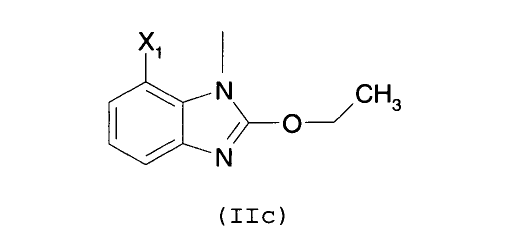 or B is the residue of formula (IIIa) when A has formula (III): 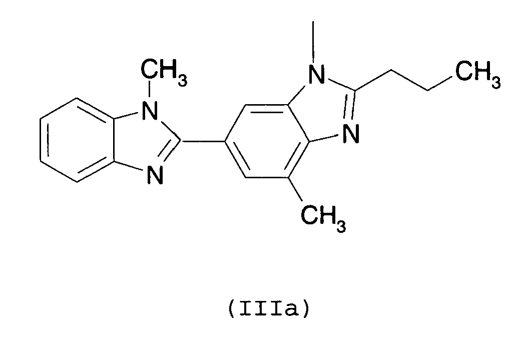 wherein both X₁ and X₂, the same or different to each other, are a group capable to bind to ~Y or ~W, selected from the group comprising -COO-; -O-; -CONH-; -OCO~; -OCOO~; -CO~;
and wherein X₁ is also, alternatively, -COOH or -H when X₂ is a group capable to bind to ~Y or ~W, selected from the group comprising -COO~; -O~;
-CONH~; -OCO~; -OCOO~; -CO~;
and wherein X₂ is also, alternatively, -COOH or -H when X₁ is a group capable to bind to ~Y or ~W, selected from the group comprising -COO~; -O~;
-CONH~; -OCO~; -OCOO~; -CO~;
Y is zero; ~(C_{n'})alkyl-, ~(C_{n'})alkyl-CO-, ~O-(C_{n'}) alkyl-O-, ~OOC-(C_{n'})alkyl-COO- ; ~O-(C_{n'})alkyl-, ~HN-(C_{n'}) alkyl-, ~OOC- (C_{n'}) alkyl-; ~(C_{n'}) alkyl-O-CO-(C_{n''}) alkyl-; ~(C_{n'}) alkyl-CO-O- (C_{n''}) alkyl- wherein (C_{n'}) alkyl and (C_{n''}) alkyl are straight or branched, and n' and n", the same or different to each other, are 0-10;
m is 0, 1-10;
W is a polysulfurated group containing 2 or more atoms of sulphur, selected from the group comprising an organic thiosulfonate moiety or a dithiole-thione derivative,
and salts thereof.

2. Compounds of general formula (I) according to claim 1, wherein W is an organic thiosulfonate moiety having the following formula:
~S-SO₂-R (V)
wherein ~S-SO₂-R is linked to A-Y~; R is a straight or branched alkyl, selected from the group comprising methyl, ethyl, propyl; alkenyl, alkinyl; alkylaryl, alkenylaryl, alkinylaryl; arylalkyl, arylalkenyl, arylalkinyl; or cycloalkyl, cycloalkenyl, cycloalkinyl; or aromatic and/or heterocyclic ring, all substituted or unsubstituted.

3. Compounds of general formula (I) according to claim 1, wherein W is a dithiole-thione derivative having formula: 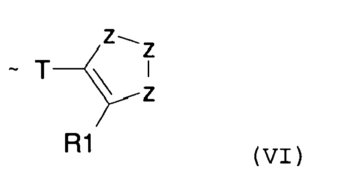 wherein
Z is S (sulphur) and at least 1 Z is is a thione C=S; and
T is:
-OOC-; or 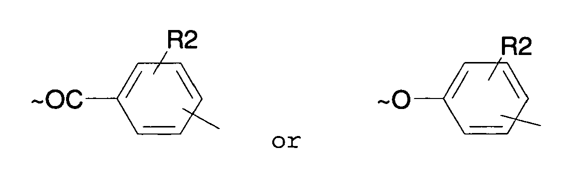 wherein
R1 is H; -COOH; -NH₂; -OH; -SH;
R2 is hydrogen; -COOH; alkyl, alkenyl, alkynyl; aryl; fluoro, chloro, bromo; hydroxyl, alkyloxy, alkenyloxy, aryloxy, acyloxy; amino, alkylamino, arylamino; thio; cyano; nitro; acyl; amido; and a 5 or 6-membered aromatic or non-aromatic ring containing 0, 1, or 2 heteroatoms selected from nitrogen; oxygen; or sulphur.

4. Salts of compounds of general formula (I) according to claim 1, wherein said salts are pharmaceutical acceptable salts of compounds of formula (I).

5. Salts according to claim 4, said salts comprising salts of compounds of formula (I) with alkaline metals and alkaline earth metals, non-toxic amines and aminoacids, inorganic acids comprising hydrochloric acid, phosphoric acid or organic acids comprising fumaric acid, citric acid, tartaric acid.

6. Compound of general formula (I) according to claim 1, that is 4-(3-thioxo-3H-1,2-dithiol-4-yl)benzoic acid 2-butyl-4-chloro-1-[*p*-(o-1*H*-tetrazol-5-ylphenyl) -benzyl]imidazole-5-methyl ester.

7. Compound of general formula (I) according to claim 1, that is 2-butyl-4-chloro-1-[(2'-(1H-tetrazol-5-yl)(1,1'-biphenyl)-4-yl)methyl]-1H-imidazole-5-carboxylic acid 4-(3H-1,2-dithiole-3-thione-5-yl)-phenyl ester.

8. Compound of general formula (I) according to claim 1, that is 2-butyl-4-chloro-1-[(2'-(1H-tetrazol-5-yl)(1,1'-biphenyl)-4-yl)methyl]-1H-imidazole-5-carboxylic acid 2-(methylsulfonylthio)ethyl ester.

9. Compound of general formula (I) according to claim 1, that is 3-(methanesulfonylsulfanyl)-propionic acid 2-butyl-4-chloro-1-[*p*-(o-1*H*-tetrazol-5-ylphenyl)-benzyl]-imidazcle-5-methyl ester.

10. Compound of general formula (I) according to claim 1, that is 4'-[(1,4'-dimethyl-2'-propyl[2,6'-bi-1*H*-benzimidazol]-1'-yl)methyl][1,1'-biphenyl]-2-carboxylic acid 4-(3H-1,2-dithiole-3-thione-5-yl)-phenyl ester.

11. Pharmaceutical composition comprising at least one compound of general formula (I) according to claims 1-10 as an active ingredient and eventually one or more pharmaceutically acceptable adjuvant(s) or carrier(s).

12. Compound of general formula (I) according to claims 1-10 for use as a medicament.

13. Use of a compound of general formula (I) according to claims 1-10 for the preparation of a pharmaceutical composition for treating cardiovascular diseases, preferably hypertension, ischemic heart disease, atherosclerosis, metabolic syndrome.

14. Use of a compound of general formula (I) according to claims 1-10 for the preparation of a pharmaceutical composition for treating cardiovascular diseases, in combination with other cardiovascular agents.

15. Process for the synthesis of compounds of general formula (I) according to claims 1-10, comprising at least a reaction between the corresponding precursor of an organic thiosulfonate or dithiol-thione, moiety W or Y-W, and an ARB compound or at least a reaction between the corresponding precursor of an organic thiosulfonate or dithiol-thione, moiety W and a corresponding precursor of an ARB modified with Y, wherein W and Y have the meaning according to claim 1.
